# EUROPEAN PATENT SPECIFICATION

(11) **EP 1 517 683 B1**
(45) Date of publication and mention of the grant of the patent: **06.06.2007**
(21) Application number: 02765720.4
(22) Date of filing: 24.07.2002
(51) Int. Cl.: A61K 31/404, A61K 9/20

(54) **SUSTAINED RELEASE TABLET CONTAINING INDAPAMIDE**
TABLETTEN MIT VERZÖGERTER WIRKSTOFFFREISETZUNG ENTHALTEND INDAPAMID
COMPRIME A LIBERATION PROLONGEE CONTENANT DE L'INDAPAMIDE

(30) Priority: 01.07.2002 PL 35482302
(43) Date of publication of application: 30.03.2005
(73) Proprietor: PLIVA Krakow, Zaklady Farmaceutyczne S.A., 31-546 Krakow (PL)
(72) Inventor: JURECZEK, Katarzyna, PL-30-718 Krakow (PL)
(74) Representative: Palmer, Jonathan R.
(86) International application number: PCT/PL2002/000056
(87) International publication number: WO 2004/002475

(56) References cited:
- EP-A- 0 519 820
- EP-A- 1 057 479
- DE-A- 3 602 304
- GB-A- 2 123 293
- DAMIEN, GERARD ET AL: "Galenic development and pharmacokinetic profile of indapamide sustained release 1.5 mg" CLINICAL PHARMACOKINETICS (1999), 37(SUPPL. 1), 13-19 , XP009004369

## Description

The present invention rotates to a sustained release tablet containing indapamide, a process of manufacturing a sustained release tablet containing indapamide, and a sustained release tablet producible by the inventive process.

Indapamide is a known medicinal product of diuretic activity administered in the therapy of primary hypertension. From a the EP 519820 B1, a sustained release matrix tablet of indapamide and a process of its manufacturing are known. Sustained release is controlled by the use of methylhydroxypropylcellulose and polyvidone, the percentages of which are from 30 to 50% and from 2 to 10%, respectively, of the total mass of the tablet. The percentages of the cellulose and polyvidone compounds provide the sustained release of indapamide in a manner that is linear for a period of at least eight hours and the release of 50% of the total quantity of indapamide within a period of from 5 to 14 hours. Additionally, the percentages of cellulose and polyvidone compounds allow the sustained release of indapamide to give blood levels in humans of from 20 to 80 ng/ml at most after administration of the tablet by the oral route. The process for the preparation of an indapamide matrix tablet according to the EP 519820 B₁ is based on the fact that both a moist granulation technique and a direct compression technique are used, comprising the steps as follows. First, indapamide, polyvidone and lactose are mixed, then moistened with an aqueous-alcoliolic solution to yield a moist mass which is then granulated, dried and then graded so as to obtain a granulate whose physical characteristics allow good filling of the moulds of a rapid compression machine. The obtained granulate is mixed with methythydoxypropylcellulose and lubricated with magnesium stearate and colloidal silica. The final step is compression of the lubricated mixture in a rotary compression machine, so as to obtain tablets having a hardness of approximately from 60 do 75 N.

The present invention provides a sustained release tablet containing indapamide, whereby the tablet contains indapamide in the amount 1.5 to 2.5. % of the total mass, of the tablet, lactose monohydrate in the amount 30 to 80 % of the total mass of the tablet, copovidon in the amount of 2 to 10 % of the total mass of the tablet, hypromellose in the amount of 20 to 65 % of the total mass of the tablet and lubricants in the amount of 0.1 to 5 % of the total mass of the tablet. This tablet shows advantages over the prior art:

Damien et al. Clin. Pharmacokinet. (1999), 37 (13-19) discloses a sustained release tablet comprising indapamide, lactose monohydrate, povidone, MHPC (= hypromellose), Mg stearat (= lubricant), and silica colloidal anhydrous (= lubricant).

According to one possible method, to produce sustained release tablets comprising indapamide, indapamide is mixed with lactose monohydrate, copovidone and then granulated with water. The granulate is then dried and homogenized with hypromellose.

Therefore, taking Damien et al. as the closest prior art, the objective problem to be solved is to provide a sustained release tablet comprising indapamide, whereby the homogenisation process which is important for the resulting controlled (sustained) drug release is optimized and thereby the production process is simplified.

This problem is solved by using copovidone as a binder as has been shown by comparative examples.

The aim of the use of copovidone is to bind all tablet components. Hypromellose modifies the release of the active ingredient, which is indapamide.

Magnesium stearate or/and anhydrous colloidal silica are preferably used as the lubricants.

Advantageously, hypromellose viscosity is between 1.000 to 20.000 cP.

According to a further aspect of the present invention, a process of manufacturing the sustained release tablet containing indapamide is provided whereby indapamide is mixed with lactose monohydrate and copovidone. Then, the mixture is moistened by purified water and granulated. The obtained granulate is then dried, cooled, mixed with hypromellose and lubricants and compressed in a tableting machine.

A further aspect of the present invention is a sustained release tablet producible by the above mentioned inventive process.

The aim of the invention was to simplify the process of manufacturing the sustained release tablet containing indapamide. Additionally, it allowed replacing alcohol with water what improved safety of the manufacturing process and reduced its impact on the environment.

Examples of the invention are presented below:

### Example I.

25 g of indapamide and 225 g of lactose monohydrate are mixed manually, the mixture is poured into a granulate-mixer together with 487 g of lactose monohydrate and is mixed for 1 minute with the main agitator speed 200 rpm.

Then 487 g of lactose monohydrate and 60 g of copovidone are added and all components are mixed for 1 minute with the main agitator speed 200 rpm and then with the main agitator speed 200 rpm and the side agitator speed 400 rpm.

To the prepared mixture 100 g of purified water are added within 1 minute with the main agitator speed 200 rpm and the side agitator speed 400 rpm. Then, the granulation process is performed for 4 minutes with the main agitator speed 400 rpm and side agitator speed 800 rpm.

Wet granulate is rubbed through the screen of 2.5 mm mesh and dried in the fluidal drier in the temperature 40°C to a humidity content below 1%. The dried granulate is sieved through the screen of 1.2 mm mesh.

In the following step the mixing of the granulate with the rest of the components is performed in the rotary mixer with a speed of 20 rpm.

Into a rotary mixer 642 g of granulate with 350 g of hypromellose are poured and mixed for 10 minutes, after which 642 g of granulate is added and is mixed for 10 minutes. Then, 350 g of hypromellose is added with 6 g of colloidal silica and is mixed for 15 minutes, after which 10 g of magnesium stearate is added and mixed for 5 minutes.

Finally, the tableting process of the prepared mixture is performed.

### Example II.

25 g of indapamide and 225 g of lactose monohydrate are mixed manually and next the mixture is poured into a granulate-mixer together with 507 g of lactose monohydrate and is mixed for 1 minute with the main agitator speed 200 rpm.

Then 507 g of lactose monohydrate and 60 g of copovidone are added and all components are mixed for 1 minute with the main agitator speed 200 rpm and 1 minute with the main agitator speed 200 rpm and the side agitator speed 400 rpm.

To the mixture obtained in such way 100 g of purified water is dosed for 1 minute with the main agitator speed 200 rpm and the side agitator speed 400 rpm.

Next the granulation process is performed for 4 minutes with the main agitator speed 400 rpm and the side agitator speed 800 rpm.

Wet granulate is rubbed through a screen of 2.5 mm mesh and is dried in the fluidal drier in the temperature 40°C to a humidity content below 1 %. The dried granulate is sieved through the screen of 1.2 mm mesh.

In the following step the mixing of the granulate with the rest of components is performed in the rotary mixer with the speed 20 rpm. Into a rotary mixer 662 g of granulate with 330 g of hypromellose is poured and mixed for 10 minutes. Then, 662 g of the granulate is added and mixed for 10 minutes, after which 330 g of hypromellose together with 6 g of colloidal silica is added and mixed for 15 minutes, and next 10 g of magnesium stearate is added and mixed for 5 minutes.

Finally, the tableting process of the prepared mixture is performed.

The invention may be used in the industrial process of manufacturing sustained release tablets containing indapamide.

## Claims

1. Sustained release tablet containing indapamide, **characterized in that** the tablet contains indapamide in the amount 1.5 to 2.5 % of the total mass of the tablet, lactose monohydrate in the amount 30 to 80 % of the total mass of the tablet, copovidon in the amount of 2 to 10 % of the total mass of the tablet, hypromellose in the amount of 20 to 65 % of the total mass of the tablet and lubricants in the amount of 0.1 to 5 % of the total mass of the tablet.

2. Tablet according to claim 1, **characterized in that** as the lubricants it contains magnesium stearate or/and anhydrous colloidal silica.

3. Tablet according to claim 1, **characterized in that** hypromellose viscosity is from 1.000 to 20.000 cP.

4. Process of manufacturing sustained release tablet containing indapamide, **characterized in that** indapamide is mixed with lactose monohydrate and copovidone and then the mixture is moisturized by purified water and its granulation is performed after which the granulate is dried, cooled, mixed with hypromellose and lubricants and compressed in tableting machine.

5. Sustained release tablet producible by the process according to claim 4.

## Patentansprüche

1. Tablette mit verzögerter Wirkstofffreisetzung umfassend Indapamid, **dadurch gekennzeichnet, dass** die Tablette Indapamid in der Menge von 1.5 bis 2.5% der Gesamtmasse der Tablette umfasst, Lactose Monohydrat in der Menge von 30 bis 80% der Gesamtmasse der Tablette, Copovidon in der Menge von 2 bis 10% der Gesamtmasse der Tablette, Hypromellose in der Menge von 20 bis 65% der Gesamtmasse der Tablette und Gleitmittel in der Menge von 0.1 bis 5% der Gesamtmasse der Tablette.

2. Tablette gemäss Anspruch 1, **dadurch gekennzeichnet, dass** sie als Gleitmittel Magnesiumstearat und/oder anhydrose kolloidale Silika umfasst.

3. Tablette gemäss Anspruch 1, **dadurch gekennzeichnet, dass** die Hypromellose Viskosität von 1.000 zu 20.000 cP beträgt.

4. Verfahren zur Herstellung einer Tablette mit verzögerter Wirkstofffreisetzung umfassend Indapamid, **dadurch gekennzeichnet, dass** Indapamid mit Laktose Monohydrat und Copovidone vermischt wird, anschliessend die Mischung mit gereinigtem Wasser befeuchtet wird und die Granulierung durchgeführt wird, wonach das Granulat getrocknet, gekühlt, mit Hypromellose und Gleitmitteln vermischt und in einer Tablettierungsmaschine gepresst wird.

5. Tablette mit verzögerter Wirkstofffreisetzung herstellbar durch das Verfahren gemäss Anspruch 4.

## Revendications

1. Comprimé à libération prolongée contenant de l'indapamide, **caractérisé en ce que** le comprimé comprend de l'indapamide dans des proportions comprises entre 1,5 et 2.5 % en poids de la masse totale du comprimé, du lactose monohydrate dans des proportions comprises entre 30 et 80 % de la masse totale du comprimé, du copovidone dans des proportions comprises entre 2 et 10 % de la masse totale du comprimé, de l'hypromellose dans des proportions comprises entre 20 et 65 % de la masse totale du comprimé et des lubrifiants dans des proportions comprises entre 0,1 et 5 % de la masse totale du comprimé.

2. Le comprimé selon la revendication 1, **caractérisé en ce que** les lubrifiants contenus sont le stéarate de magnésium ou/et le silice colloïdale anhydre.

3. Le comprimé selon la revendication 1, **caractérisé en ce que** la viscosité de l'hypromellose est comprise entre 1.000 et 20.000 cP.

4. Le procédé d'obtention d'un comprimé à libération prolongée contenant de l'indapamide, **caractérisé en ce que** l'indapamide est mélangé au lactose monohydrate et au copovidone et le mélange est alors humidifié avec de l'eau purifiée et sa granulation est effectuée après qoui le granulé est séché, refroidi, mélangé à l'hypromellose et aux lubrifiants et compressé dans un appareil de pastillage.

5. Comprimé à libération prolongée qui peut être obtenu suivant le procédé selon la revendication 4.
